# EUROPÄISCHE PATENTSCHRIFT

(11) **EP 2 340 125 B1**
(45) Veröffentlichungstag und Bekanntmachung des Hinweises auf die Patenterteilung: **23.03.2016**
(21) Anmeldenummer: 09741185.4
(22) Anmeldetag: 23.10.2009
(51) Int. Cl.: A61B 17/00, B05B 7/04, B05B 7/24, B05C 17/005, B05B 7/08

(54) **SPRÜHKOPF UND SPRÜHVORRICHTUNG MIT DRUCKGASLEITUNG**
SPRAY HEAD AND SPRAYING DEVICE HAVING PRESSURIZED GAS LINE
TÊTE DE PULVÉRISATION ET DISPOSITIF DE PULVÉRISATION AVEC CONDUIT DE GAZ SOUS PRESSION

(30) Priorität: 30.10.2008 CH 17062008
(43) Veröffentlichungstag der Anmeldung: 06.07.2011
(73) Patentinhaber: Medmix Systems AG, 6343 Rotkreuz (CH)
(72) Erfinder: BREM, William, CH-5630 Muri (CH)
(74) Vertreter: Detken, Andreas
(86) Internationale Anmeldenummer: PCT/CH2009/000341
(87) Internationale Veröffentlichungsnummer: WO 2010/048734

(56) Entgegenhaltungen:
- WO-A-97/48496
- US-A- 6 062 492
- US-A1- 2003 226 910

## Beschreibung

Die vorliegende Erfindung betrifft eine Sprühvorrichtung und einen Sprühkopf für eine Sprühvorrichtung zum Versprühen wenigstens einer Substanz, bzw. einer Komponente durch ein Druckgas, insbesondere einen Sprühkopf und eine Sprühvorrichtung, bei welchen einzelne Komponenten nach dem Austritt aus dem Sprühkopf mittels des Druckgases vermischt werden.

### Stand der Technik

Aus dem Stand der Technik sind Sprühvorrichtungen bekannt, die z. B. bei Behandlungsverfahren in der Medizinaltechnik verwendet werden. Mit Hilfe der Sprühvorrichtungen kann z. B. Klebstoff, wie etwa Vibrin oder Thrombin, berührungslos auf eine Behandlungsfläche aufgebracht werden. Solche Verfahren werden beispielsweise bei der Wundbehandlung oder beim Stoppen von Blutungen eingesetzt. Die mit der Sprühvorrichtung applizierten Substanzen werden oftmals in Form getrennter Komponenten z. B. in einer Doppelkartusche oder Doppelspritze gelagert und erst bei der Anwendung werden die Komponenten miteinander vermischt. Das Mischen der Komponenten kann unmittelbar nach dem Austritt der einzelnen Komponenten aus einem Sprühkopf der Sprühvorrichtung bei der Zerstäubung der einzelnen Komponenten erfolgen. Es ist auch möglich, die Komponenten in einem Mischkanal innerhalb der Vorrichtung zu Mischen und sofort zu versprühen. Allerdings können dabei Verklebungen oder Verschmutzungen innerhalb der Sprühvorrichtung auftreten. Die Komponenten werden aus dem Sprühkopf ausgetragen und durch einen gleichzeitig auf die Komponenten gerichteten Gasstrom in einzelne Tröpfchen zerstäubt. Der Gasstrom transportiert die gemischten Komponenten auf eine Behandlungsstelle. Dabei ist darauf zu achten, dass das Sprühbild eine gleichmässig feine Tropfengrösse und eine möglichst gleichmässige Durchmischung der Komponenten aufweist. Für einige Anwendungen ist es notwendig, die vermischten Komponenten in einem möglichst engen Fokus zu transportieren. Weiter ist es wünschenswert, mit möglichst wenig Druckgas auch hochviskose Komponenten versprühen zu können.

Aus der US 5,605,541 ist beispielweise eine Sprühvorrichtung für einen Vibrinkleber bekannt, bei dem die einzelnen Komponenten und ein Gas in getrennten Kanälen in einen Sprühkopf geführt werden. Dabei ist der Gaskanal zentriert in dem Sprühkopf angeordnet und die Komponentenkanäle verlaufen ringförmig um den Gaskanal. Die Kanäle münden in einer gemeinsamen Ebene an der Spritze des Sprühkopfes, so dass beim Austragen der Komponenten und des Gases eine Mischung der Komponenten entsteht. Bei dieser Sprühvorrichtung treten die Komponenten ringförmig über einen grossen Bereich aus dem Sprühkopf aus. Es erfolgt keine koordinierte Abgabe der Komponenten und des Druckgases in eine bestimmte Richtung und der Abstand zwischen dem Druckgaskanal und dem aussen liegenden Komponentenkanal ist sehr gross, so dass ein vollständiges Erfassen der Komponenten von dem Druckgas kaum gewährleistet ist.

In der US 6,062,492 wird ein Sprühkopf gezeigt, der am Ende einer Mischdüse angeordnet ist. In die Mischdüse werden aus zwei unterschiedlichen Leitungen zwei unterschiedliche Komponenten eingeführt und innerhalb der Mischdüse vermischt. Der Sprühkopf an der Spitze der Mischdüse umfasst eine Kappe, die um ein Mischrohr angeordnet ist. Zwischen dem Mischrohr und der Kappe verbleibt ein Ringkanal der an der Spitze des Sprühkopfes neben einem Auslass des Mischrohrs endet. Die aus dem Ringkanal strömende Druckluft verwirbelt die bereits vermischten Komponenten.

In der US 2003/002610 ist ebenfalls ein Sprühkopf gezeigt, der zum Versprühen eines Mischprodukts aus einer Mischdüse dient. Der Sprühkopf weist eine Kappe auf, die auf die Mischdüse aufgesetzt werden kann. Die Kappe bildet einen Ringkanal um die Sprühdüse, in den ein Druckgas eingeleitet werden kann. An der Spitze des Sprühkopfes ist in die Kappe ein Kanalelement eingesetzt, das am Aussenumfang Rillen aufweist, welche spiralförmig oder wendelartig um den Umfang der Sprühdüse verlaufen. Das Druckgas strömt entlang von diesen Rillen und verlässt den Sprühkopf in der gleichen Ebene, in welcher der Sprühdüsenauslass endet.

Die WO 97/48496 zeigt ein weiteres Beispiel eines Sprühkopfes, der einen Komponentenkanal aufweist, um den ein Ringkanal für ein Druckgas angeordnet ist.

Es ist daher eine Aufgabe der vorliegenden Erfindung, einen Sprühkopf und eine Sprühvorrichtung zu schaffen, die ein gleichmässiges Mischen von Komponenten und eine gleichmässig feine Tropfenbildung ermöglichen, eine gezielte und enge Fokussierung der Komponenten und der Mischung vorsehen, nur wenig Druckgas zum Versprühen benötigen, ein Verkleben oder Verschmutzen des Sprühkopfes vermeiden und auch für hochviskose Komponenten geeignet sind.

Diese Aufgabe wird durch einen Sprühkopf nach Patentanspruch 1 sowie durch eine Sprühvorrichtung nach Patentanspruch 13 erfüllt. Vorteilhafte Ausgestaltungen des Sprühkopfes und der Sprühvorrichtung gehen aus den Unteransprüchen hervor.

### Darstellung der Erfindung

Gemäss der vorliegenden Erfindung wird ein Sprühkopf für eine Sprühvorrichtung zum Versprühen wenigstens einer Substanz, bzw. einer Komponente vorgesehen. Der Sprühkopf umfasst wenigstens einen Substanz-, bzw. Komponentenkanal, in dem die Substanz, bzw. die Komponente geführt wird. Vorzugsweise sind jeweils zwei Komponentenkanäle für jeweils eine Sprühkomponente vorgesehen. Die Komponentenkanäle weisen Komponentenauslässe auf, die nebeneinander angeordnet sind und aus einer Spitze des Sprühkopfes münden. Die Komponentenauslässe sind vorzugsweise dicht nebeneinander an der Sprühkopfspitze vorgesehen. Beispielsweise entspricht der Abstand zwischen den beiden Komponentenauslässe ungefähr der Dimension ihrer Durchmesser. Die Komponentenkanäle können dabei weiter auseinander liegend vorgesehen werden. Vorzugsweise werden die Komponentenkanäle innerhalb des Sprühkopfes parallel nebeneinander in Sprührichtung geführt. Die Komponentenkanäle können aber auch voneinander unabhängig durch den Sprühkopf geführt werden, solange ihre Komponentenauslässe an der Sprühkopfspitze nah beieinander zu liegen kommen. Die unterschiedlichen Sprühkomponenten werden aus Behältern, wie etwa einer Doppelkartusche oder einer Doppelspritze, in die Komponentenkanäle eingeleitet.

Der Sprühkopf umfasst weiter einen Ringkanal für ein Druckgas, der die Komponentenkanäle in Längsrichtung zumindest teilweise umgibt und an der Spitze des Sprühkopfes aus dem Sprühkopf mündet. Dabei ist es ausreichend, wenn das Druckgas die Komponentenkanäle in einem Bereich an der Sprühkopfspitzen und nahe der Sprühkopfspitze umgibt. Es ist aber auch möglich, dass der Ringkanal sich in Längsrichtung entlang der Komponentenkanäle erstreckt. Vorzugsweise werden die Komponentenkanäle konzentrisch von dem Ringkanal umgeben. Es ist darauf zu achten, dass der Ringkanalauslass an der Sprühkopfspitze nahe bei den Komponentenauslässen zu liegen kommt. Der Ringkanal kann hierfür vorteilhafterweise verjüngend in Richtung der Komponentenauslässe ausgebildet sein. Durch die konzentrische Anordnung des Ringkanalauslasses sind die Komponentenkanäle jeweils gleichweit von einem Bereich des Ringkanals beabstandet.

Es ist ein Druckgaszufuhrkanal zum Einleiten von Druckgas in den Ringkanal vorgesehen. Der Druckgaszufuhrkanal ist vorzugsweise seitlich an dem Sprühkopf vorgesehen und weist einen Anschluss für eine Vorrichtung zur Druckgaserzeugung auf. Es ist auch möglich, den Druckgaszufuhrkanal parallel zu dem Ringkanal vorzusehen, so dass das Druckgas nicht seitlich in den Ringkanal eingeführt wird, sondern in Längsrichtung der Sprührichtung. Zur Druckgaserzeugung können beispielsweise Gaspatronen oder Pumpen verwendet werden. Für die meisten Anwendungen kann Druckluft als Druckgas verwendet werden. Bei der Anwendung des Sprühkopfes, bzw. der Sprühvorrichtung, werden die Sprühkomponenten aus den Komponentenkanälen und gleichzeitig das Druckgas aus dem Ringkanal ausgetragen.

Gemäss der vorliegenden Erfindung weist der Ringkanal mehrere Stege auf, die den Ringkanal wenigstens im Bereich der Spitze des Sprühkopfes in voneinander getrennte Druckgasauslasskanäle unterteilen. Der Ringkanal wird somit in einzelne Druckgasauslasskanäle unterteilt, die ringförmig um die Komponentenkanäle angeordnet sind. Die Stege können unterschiedliche Länge und Breite aufweisen, so dass verschiedene Druckgasauslasskanäle ausgebildet werden können. Die einzelnen Druckgasauslasskanäle schliessen sich unmittelbar an den Ringkanal an. Ein durch den Druckgaszufuhrkanal eingeleitetes Druckgas fliesst durch den Ringkanal und verteilt sich auf die einzelnen Druckgasauslasskanäle. Die Stege können in Längsrichtung in Richtung der Spitze derart angeordnet, z. B. verbreitert, sein, dass sich die Druckgasauslasskanäle in Richtung der Spitze verjüngen. Weiter kann durch unterschiedliche Breite der Stege ein unterschiedlicher Abstand der Druckgasauslasskanäle an der Sprühkopfspitze und es können Druckgasauslasskanäle mit unterschiedlichem Durchmesser erzielt werden.

Durch einen erfindungsgemässen Sprühkopf mit einem Ringkanal, der durch mehrere Stege in getrennte Druckgasauslasskanäle unterteilt ist, kann die zur Zerstäubung der Komponenten erforderliche Gasmenge verringert werden. Der Druckgasstrom kann gezielt in definierter Weise relativ zu den Komponentenkanälen angeordnet werden. Weiter kann die Verteilung der Menge des Druckgases an eine entsprechende Komponente oder eine Komponentenkombination angepasst werden. Dadurch wird eine gleichmässige Durchmischung der Komponenten und eine Zerstäubung mit gleichmässig feiner Tropfengrösse möglich. Die Stege innerhalb des Ringkanals vereinfachen die Kanalführung innerhalb des Sprühkopfes. Es ist nicht notwendig, einzelne Druckgaskanäle durch den gesamten Sprühkopf zu führen.

In einer bevorzugten Ausführungsform eines Sprühkopfes und einer Sprühvorrichtung nach der vorliegenden Erfindung sind zwei Komponentenkanäle in einem Gehäuse, bzw. einem Gehäusebauteil, ausgebildet. Das Gehäuse ist vorzugsweise länglich und läuft im Bereich der Sprühkopfspitze spitz zu. Es kann z. B. zylindrisch oder oval ausgestaltet sein. Die Komponentenkanäle verlaufen in Längsrichtung des Gehäuses parallel nebeneinander. Sie münden an dem spitzen Ende dicht nebeneinander an Komponentenauslässen aus dem Gehäuse. An dem gegenüberliegenden Ende münden die Komponentenkanäle beispielsweise in Einlassöffnungen, durch welche eine Komponente aus einem Behälter in den jeweiligen Komponentenkanal eingeführt werden kann. Die Behälter können z. B. durch eine Doppelkartusche gegeben sein, die zwei Behälter für jeweils eine Komponente aufweist, wobei die jeweiligen Behälterauslässe in die Öffnungen am Gehäuse münden. Die Sprühvorrichtung weist zum Austragen der Komponenten eine Austragvorrichtung auf, die z. B. durch eine Doppelkolbenstange gegeben ist, wobei je ein Kolben der Doppelkolbenstange in jeweils einen der Behälter ragt. Durch Vorschub der Doppelkolbenstange werden die Komponenten aus den Behältern durch die Behälterauslässe ausgetragen und in die Komponentenkanäle des Sprühkopfes eingeführt.

An dem Sprühkopf ist eine Kappe oder Hülse vorgesehen, die auf das Gehäuse mit den Komponentenkanälen aufgesetzt werden kann. Dabei wird zwischen einer Aussenwand des Gehäuses und einer Innenwand der Kappe der Ringkanal für das Druckgas ausgebildet. Hierfür schliesst die Kappe an einem Ende dicht mit dem Gehäuse ab und am anderen Ende ist eine Öffnung vorgesehen, die zur Ausbildung eines Druckgasauslasses zwischen dem Gehäuse und der Kappe vorgesehen ist. Vorzugsweise ragt die Gehäusespitze mit den Komponentenauslässen in die Öffnung der Kappe hinein, so dass die Komponentenauslässe seitlich neben den Druckgasauslasskanäle zu liegen kommen oder auch in Sprührichtung über die Druckgasauslasskanäle aus der Kappe hervorstehen.

In einer Ausführungsform ist die Kappe von dem Gehäuse abnehmbar und der Druckgaszufuhrkanal ist an der Kappe vorgesehen, beispielweise durch einen seitlichen Druckgasanschluss an der Kappe. Die Kappe kann somit für verschiedene Sprühköpfe verwendet werden, d. h. sie kann auf Gehäuse von verschiedenen Sprühköpfen aufgesteckt werden. Grundsätzlich kann die Kappe aber auch fest an dem Gehäuse angebracht werden, so dass sie nicht versehentlich vom Gehäuse getrennt werden kann.

Vorzugsweise sind die Stege zur Ausbildung der Druckgasauslasskanäle im Ringkanal an der Aussenwand des Gehäuses radial abstehend angeordnet. Wird die Kappe auf das Gehäuse aufgesetzt, liegen die Stege an der Innenwand der Kappe an, so dass voneinander getrennte Druckgasauslasskanäle gemäss der vorliegenden Erfindung ausgebildet werden. Es ist jedoch auch möglich, die Stege an der Innenwand der Kappe radial nach innen abstehend vorzusehen, so dass die Stege bei aufgesetzter Kappe an der Aussenwand des Gehäuses zu liegen kommen. Auch eine Kombination aus diesen Möglichkeiten ist denkbar.

Bei einer vorteilhaften Ausgestaltung der vorliegenden Erfindung stehen die Komponentenauslässe an der Sprühkopfspitze in Sprührichtung über die Druckgasauslasskanäle hervor. Dadurch wird sichergestellt, dass eine Vermischung der Sprühkomponenten ausserhalb des Sprühkopfes erfolgt und somit der Sprühkopf vor einem Verkleben durch ein zu frühes Mischen der Komponenten gesichert wird. Vorzugsweise sind zwei Komponentenauslässe vorgesehen, die jeweils an einer Fläche der Sprühkopfspitze austreten, wobei die jeweiligen Flächen zueinander einen Winkel einschliessen, so dass die Komponentenauslässe schräg zur Sprührichtung nach aussen gerichtet sind. Bei dieser Anordnung weisen die Komponentenauslässe in unterschiedliche Richtungen, die vorzugsweise zu einander entgegengesetzt sind. Dadurch kann abermals ein vorzeitiges Vermischen und damit ein Verkleben des Sprühkopfes verhindert werden. Die Austrittsflächen der Komponentenauslässe sind demnach pfeilförmig aufeinander zulaufend ausgebildet, so dass sie an der Sprühkopfspitze eine Kante ausbilden, welche den vordersten Punkt der Spitze bildet. Der vorderste Bereich des Gehäuses zwischen den Komponentenauslässen bildet zudem ein Abschirmmittel, dass die beiden Komponentenauslässe von einander abschirmt. Beim Austragen der Komponenten aus den Komponentenauslässen wird dadurch sichergestellt, dass die Komponenten nicht schon vor ihrer Zerstäubung miteinander in Kontakt treten. Die Flächen sind vorzugsweise in einem Winkel von 90 Grad zueinander angeordnet. Sie können aber auch in einem spitzeren oder stumpferen Winkel vorgesehen werden.

Die Stege innerhalb des Ringkanals sind vorzugsweise derart angeordnet, dass die Druckgasauslasskanäle neben einer Verbindungslinie der beiden Komponentenauslässe angeordnet sind. Demnach wird das austretende Druckgas nicht entlang der Flächen unmittelbar auf die Komponentenauslässe gerichtet, sondern strömt seitlich in den Zerstäubungsbereich vor den Komponentenauslässen ein. Dadurch wird verhindert, dass durch einen starken Druckgasstrom durch eine Vakuumbildung die Komponenten aus den Komponentenkanälen herausgesogen werden. Erst wenn die Austragvorrichtung zum Austragen der Komponenten aus den Behältern und somit aus den Komponentenauslässen betätigt wird, werden die Komponenten in den Zerstäubungsbereich vor der Sprühkopfspitze ausgetragen. Ein unkontrolliertes Austragen der Komponenten und somit ein unabsichtliches Mischen der Komponenten wird dadurch verhindert. Um die Druckgasauslasskanäle gezielt derart neben den Komponentenauslässen anzuordnen, dass das Druckgas nicht direkt über die Auslässe strömt, können die Stege im Ringkanal entsprechend angeordnet werden. Beispielsweise werden Stege jeweils auf der Verbindungslinie der beiden Kanäle vorgesehen.

Bei einer anderen Ausführungsform der vorliegenden Erfindung ist in dem Sprühkopf ein Substanzkanal, bzw. ein Mischkanal für mehrere Komponenten, vorgesehen, dessen Auslass aus der Sprühkopfspitze mündet und der von einem erfindungsgemässen Ringkanal in längs Richtung zumindest teilweise umgeben ist. Vorzugsweise münden in den Substanzkanal, bzw. Mischkanal, zwei Behälterauslässe von zwei Behältern einer Doppelkartusche, wobei in den beiden Behältern der Doppelkartusche jeweils unterschiedlich Komponenten vorgesehen sind. Bei der Ausgestaltung als Mischkanal sind Mischelemente zwischen der Einmündung der Behälterauslässe der Doppelkartusche und dem Auslass an der Sprühkopfspitze vorgesehen. Die Mischelemente dienen zum Mischen der einzelnen Komponente aus der Doppelkartusche unmittelbar bevor die Mischung aus der Sprühvorrichtung ausgetragen wird. In einer besonders bevorzugten Ausführungsform teilt sich der eine Mischkanal an der Spitze des Sprühkopfes in zwei nebeneinander angeordnete Auslässe. Vorzugsweise sind die beiden Auslässe jeweils an einer Fläche der Sprühkopfspitze vorgesehen, wobei die Flächen zueinander einen Winkel einschliessen, so dass die Auslässe schräg zur Sprührichtung nach aussen vorgesehen sind, wie vorher bereits beschrieben wurde.

Der Ringkanal für das Druckgas, der den Substanzkanal, bzw. Mischkanal mit den Mischelement, in Längsrichtung zumindest teilweise umgibt weist erfindungsgemäss mehrere Stege auf, die den Ringkanal in voneinander getrennte Druckgasauslasskanäle unterteilen. Wie vorher beschrieben, ist es dabei vorteilhaft, wenn die Druckgasauslasskanäle nicht direkt auf die gewinkelt angeordneten Flächen mit den Komponentenauslässen gerichtet sind, sondern mittels der Stege seitlich davon vorgesehen sind.

Es wird darauf hingewiesen, dass die Anordnung der Komponentenauslässe an zueinander gewinkelt angeordneten Flächen der Sprühkopfspitze, derart dass die Komponentenauslässe relativ zu den Komponentenkanälen schräg zur Sprührichtung nach aussen weisen, unabhängig von dem Vorhanden sein eines Ringkanals mit Stegen, wie er oben beschrieben ist, eine vorteilhafte Ausgestaltung eines Sprühkopfes für eine Sprühvorrichtung zum Versprühen einer Substanz, bzw. einer Komponente darstellt. Es bleibt damit vorbehalten, unabhängige Ansprüche auf einen Sprühkopf für eine Sprühvorrichtung zu richten, der wenigstens einen Substanz-, bzw. Komponentenkanal aufweist, wobei der, bzw. die Komponentenkanäle an einer Spitze des Sprühkopfes in wenigstens zwei Komponentenauslässe münden. Der Sprühkopf umfasst wenigstens einen Druckgaskanal, der, bzw. die, an der Spitze des Sprühkopfes aus dem Sprühkanal mündet, bzw. münden, und einen Druckgaszufuhrkanal zum Einleiten von Druckgas in den oder die Druckgaskanäle. Wesentlich ist dabei, dass die Komponentenauslässe jeweils an einer Fläche des Sprühkopfes austreten, wobei die Flächen zueinander einen Winkel derart einschliessen, dass die Komponentenauslässe relativ zur Sprührichtung schräg nach aussen gerichtet sind. Das heisst, die Ausgänge der Komponentenkanäle sind schräg radial nach aussen ausgerichtet. Die jeweiligen Flächen sind demnach pfeilförmig aufeinander zulaufend vorgesehen. Vorzugsweise stehen die Komponentenauslässe in Sprührichtung über die Auslässe der Druckgaskanäle hervor und die Druckgasauslässe sind neben einer Verbindungslinie von zwei Komponentenauslässen angeordnet. Beispielsweise im Fall von einem Substanzkanal, der sich durch den Sprühkopf erstreckt, kann sich dieser an der Sprühkopfspitze in zwei Komponentenauslässe aufteilen. Im Fall von zwei getrennt nebeneinander verlaufenden Komponentenkanälen können diese jeweils in eine der gewinkelt zueinander vorgesehenen Flächen an der Sprühkopfspitze münden.

### Kurze Beschreibung der Zeichnungen

Bevorzugte Ausführungsformen der Erfindung werden anhand der Zeichnung dargestellt, die in keiner Weise einschränkend auszulegen ist. Auf den Figuren der Zeichnung ersichtlich werdenden Merkmale sollen als zur Offenbarung der Erfindung gehörend verstanden werden. In der Zeichnung stellen dar:
- Figur 1:: Längsschnitt einer Sprühvorrichtung mit einem Sprühkopf gemäss einer ersten Ausführungsform nach der vorliegenden Erfindung,
- Figur 2:: Längsschnitt des Sprühkopfes der Sprühvorrichtung nach der vorliegenden Erfindung,
- Figur 3:: dreidimensionale Explosionsdarstellung des Sprühkopfes nach der Erfindung,
- Figur 4a:: Längsschnitt entlang einer ersten Ebene durch eine Sprühkopfspitze,
- Figur 4b:: Längsschnitt entlang einer zweiten Ebene durch die Sprühkopfspitze,
- Figur 4c:: dreidimensionale Darstellung der Sprühkopfspitze,
- Figur 5a:: Frontansicht einer Sprühkopfspitze nach Figur 4c mit zwei Austrittsflächen,
- Figur 5b:: Frontansicht einer Sprühkopfspitze mit drei Austrittsflächen,
- Figur 5c:: Frontansicht einer Sprühkopfspitze mit vier Flächen,
- Figur 5d:: Frontansicht einer Sprühkopfspitze mit vier Austrittsflächen,
- Figur 6:: dreidimensionale Darstellung einer Sprühkopfspitze mit einer weiteren Ausführungsform eines Abschirmmittels,
- Figur 7:: Längsschnitt durch einen Sprühkopf gemäss einer zweiten Ausführungsform nach der Erfindung,
- Figur 8a:: Längsschnitt entlang einer ersten Ebene durch eine Sprühkopfspitze nach der zweiten Ausführungsform und
- Figur 8b:: Längsschnitt entlang einer zweiten Ebene der Sprühkopfspitze nach der zweiten Ausführungsform.

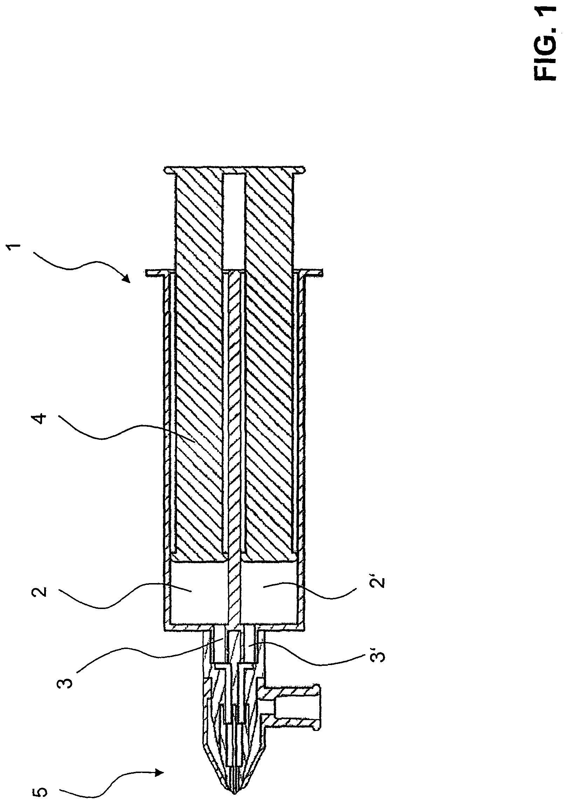

In Figur 1 ist eine Sprühvorrichtung und ein Sprühkopf gemäss der vorliegenden Erfindung in einer ersten Ausführungsform gezeigt. Die Sprühvorrichtung umfasst eine Doppelspritze 1 mit zwei Behältern 2 und 2', in welchen jeweils eine Komponente vorgesehen ist, die mit der Sprühvorrichtung versprüht werden soll. In den Behältern sind beispielsweise zwei unterschiedliche Komponenten, die durch Mischung einen Vibrinkleber bilden. Die Behälter 2 und 2' weisen jeweils einen Behälterauslass 3 und 3' auf. Die Behälter 2 und 2' weisen an dem Ende, das den Behälterauslässen 3 und 3' gegenüberliegt, eine Austragvorrichtung in Form eines Doppelkolbens 4 auf. Durch Vorschub des Doppelkolbens 4 in das Innere der Behälter 2 und 2' werden die Komponenten aus dem Inneren der Behälter durch die Behälterauslässe 3 und 3' ausgetragen. An die Behälterauslässe schliesst sich ein Sprühkopf 5 gemäss der vorliegenden Erfindung an.

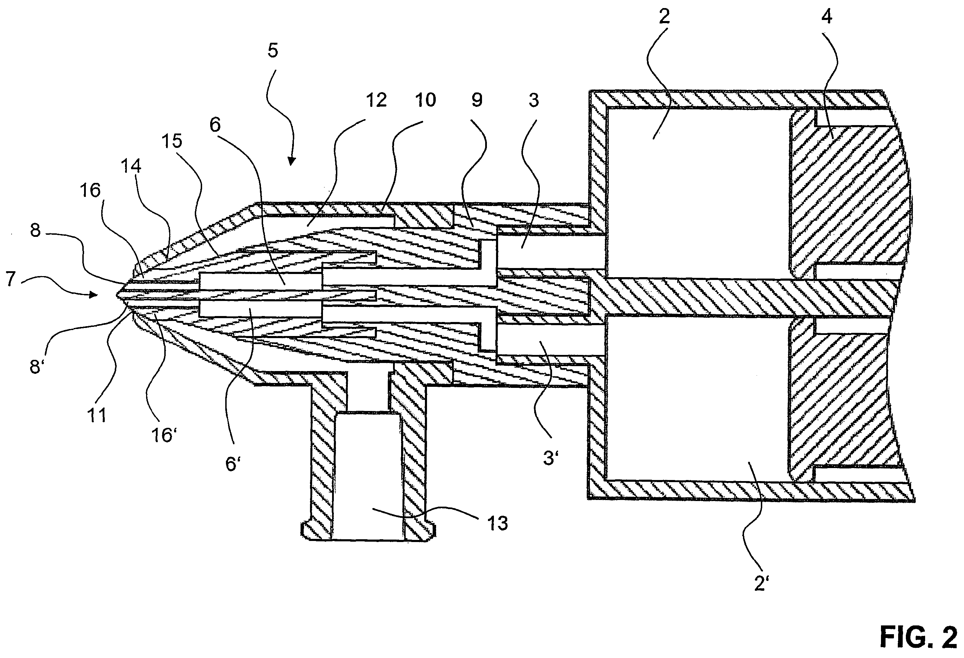

Der Sprühkopf 5 ist in Figur 2 im Detail dargestellt. Im Inneren des Sprühkopfes verlaufen zwei parallel zueinander angeordnete Komponentenkanäle 6 und 6' nebeneinander von einem Einlassende des Sprühkopfes bis zu einer Sprühkopfspitze 7, aus der zwei Komponentenauslässe 8 und 8' der Komponentenkanäle 6 und 6' münden. Am Einlassende weist der Sprühkopf 5 Aufnahmeöffnungen zur Aufnahme der Behälterauslässe 3 und 3' der Doppelspritze auf. Die Komponentenkanäle 6 und 6' schliessen sich somit mit einer Flüssigkeitsverbindung an die Behälterauslässe an, so dass beim Austragen der Komponenten aus den Behältern 2 und 2' die Komponenten durch die Behälterauslässe 3 und 3' in die Komponentenkanäle 6 und 6' ausgetragen werden. Die Komponentenkanäle sind in einem Gehäusebauteil 9 untergebracht, in dem auch die Aufnahmeöffnungen zur Aufnahme der Behälterauslässe vorgesehen sind. Die Behälterauslässe 3 und 3' können in die Öffnungen des Gehäusebauteils 9 eingeführt werden. Dabei ist es möglich, eine Sicherungseinrichtung z. B. in Form einer Schnappverbindung vorzusehen, so dass das Gehäusebauteil 9 des Sprühkopfes 5 sicher an den Behälterauslässen 3 und 3' der Doppelkartusche 1 befestigt ist.

Über dem Gehäusebauteil 9 ist eine Kappe 10 aufgesetzt, die an ihrem übergestülpten Ende dicht mit dem Gehäusebauteil 9 abschliesst. Hierfür kann beispielsweise eine formschlüssige oder kraftschlüssige Verbindung verwendet werden. Es ist auch möglich, zwischen diesem Ende der Kappe 10 und dem Gehäusebauteil 9 eine Dichtung, wie etwa einen Dichtring, vorzusehen. Am gegenüberliegenden Ende der Kappe 10 weist die Kappe eine Öffnung 11 auf, aus der der Bereich des Gehäusebauteils 9, der die Sprühkopfspitze 7 bildet, aus der Kappe 10 hervortritt. Der Bereich des Gehäusebauteils 9 der Sprühkopfspitze 7 tritt dabei derart weit aus der Kappe 10 hervor, dass die Komponentenauslässe 8 und 8' ausserhalb der Kappe angeordnet sind.

Zwischen der Kappe 10 und dem Gehäusebauteil 9 ist ein Ringkanal 12 ausgebildet, der zur Aufnahme eines Druckgases vorgesehen ist. An einer Seite weist die Kappe 10 einen Druckgaszufuhrkanal 13 auf, durch den Druckgas in den Ringkanal 12 eingeleitet werden kann. An den Druckgaszufuhrkanal 13 können herkömmliche Druckgaseinrichtungen, wie z. B. Patronen, angeschlossen werden. Wenn die Kappe 10 auf das Gehäusebauteil 9 aufgesetzt ist, wird der Ringkanal 12 zwischen einer Innenwand 14 der Kappe und einer äusseren Umfangswand 15 des Gehäusebauteils 9 ausgebildet. Das Gehäusebauteil 9 und die Kappe 10 sind in Richtung der Sprühkopfspitze 7 verjüngt ausgebildet. Der Sprühkopf 5 verläuft daher in Richtung der Sprühkopfspitze 7 spitz zu. Die Form der Kappe 10 ist an die Aussenumfangswand 15 des Gehäusebauteils 9 derart angepasst, dass der Abstand zwischen der Gehäuseaussenwand 15 und der Kappeninnenwand 14 gering ist, so dass der Ringkanal 12 ein kleines Volumen aufweist.

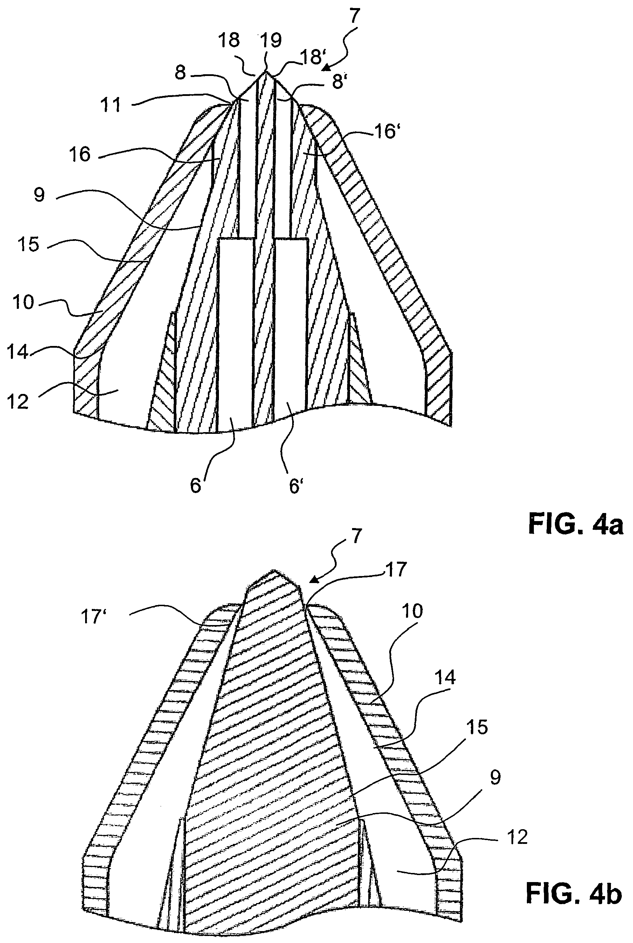

Im Bereich der Sprühkopfspitze 7 weist das Gehäusebauteil 9 auf seiner äusseren Umfangswand 15 mehrere Stege 16 und 16' auf. Die Stege berühren die Innenwand 14 der Kappe 10, wenn die Kappe 10 auf das Gehäusebauteil 9 aufgesetzt ist. Zwischen den Stegen 16, 16' bilden sich Druckgasauslasskanäle 17 und 17' aus, die sich an den Ringkanal 12 anschliessen und voneinander getrennt sind (siehe Figur 4b).

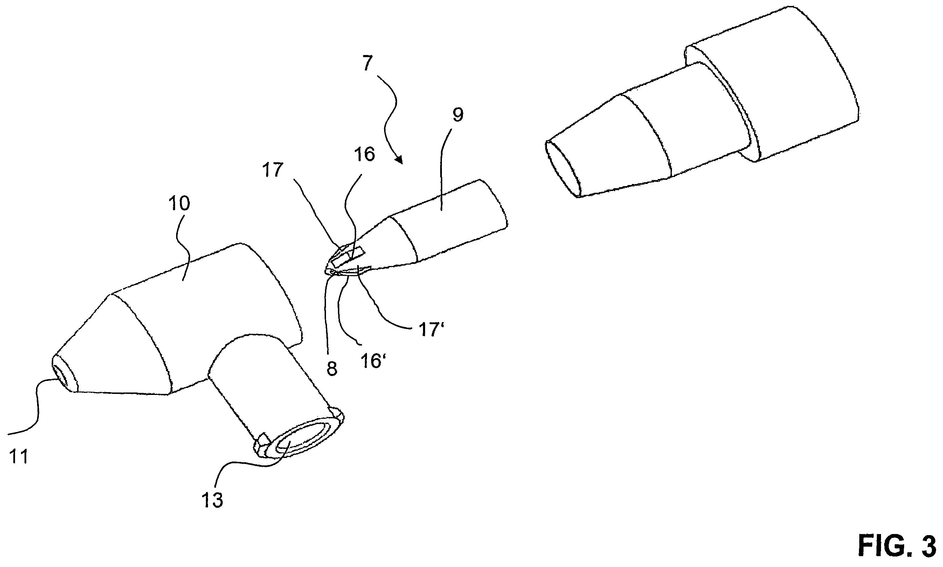

In Figur 3 sind die einzelnen Elemente des Sprühkopfes 5 in einer Explosionsdarstellung gezeigt. Daraus ist ersichtlich, dass die Kappe 10 einen zylindrischen Teil aufweist, an den sich ein kegelförmiger Teil anschliesst, an dessen Spitze die Öffnung 11 vorgesehen ist. Seitlich an dem zylindrischen Teil ist der Druckgaszufuhrkanal 13 angeordnet. Das Gehäusebauteil 9 ist in der Darstellung zweiteilig gezeigt. Zur Ausbildung des Gehäusebauteils 9 werden die beiden Teile zusammengesetzt. Die zweiteilige Form des Gehäusebauteils erleichtert die Herstellung des Bauteils. Das Bauteil weist demnach einen zylindrischen Teil auf, durch welchen die Komponentenkanäle verlaufen. An einem Ende ist das Gehäusebauteil kegelartig ausgebildet. Im Spitzenbereich des Kegels ist die Sprühkopfspitze 7 ausgebildet. Im Bereich der Sprühkopfspitze 7 weist das Gehäusebauteil 9 auf seiner Aussenumfangswand Stege 16 und 16' auf, die in Längsrichtung des Gehäusebauteils verlaufen. Wird die Kappe 10 auf das Gehäusebauteil 9 aufgesetzt, liegen die äusseren Flächen der Stege 16, 16' an der Innenwand 14 der Kappe an. Zwischen den Stegen 16, 16' bilden sich dann die Druckauslasskanäle aus 17, 17' aus.

In den Figuren 4a und 4b sind Schnitte durch die Sprühkopfspitze des Sprühkopfes 5 gezeigt. In Figur 4a verläuft der Schnitt durch zwei sich gegenüberliegende Stege 16 und 16', die an der Gehäuseaussenwand 15 vorgesehen sind. Der Ringspalt 12, der sich zwischen der Kappeninnenwand 14 und der Gehäuseaussenwand 15 ausbildet, ist im Bereich der Sprühkopfspitze 7 verschlossen, da die Stege 16, 16' des Gehäusebauteils 9 an der Kappeninnenwand 14 anliegen.

Die Komponentenauslässe 8, 8' der Komponentenkanäle 6, 6' münden ausserhalb der Kappe 10 aus dem Gehäusebauteil 9, da der Spitzenbereich des Gehäusebauteils 9 durch die Öffnung 11 aus der Kappe 10 hervorsteht. Die Komponentenauslässe 8 und 8' sind jeweils an einer Fläche 18, 18' des Gehäusebauteils 9 des Sprühkopfes vorgesehen, die zueinander einen Winkel einschliessen, so dass die Komponentenauslässe 8, 8' relativ zu den Komponentenkanälen 6 und 6' schräg zur Sprührichtung nach aussen ausgerichtet sind. Das heisst, dass die Flächen 18, 18' der Komponentenauslässe 8, 8' derart schräg zur Längsachse der Komponentenkanäle 6, 6' vorgesehen sind, dass die Komponentenauslässe 8 und 8' eine Komponente zur Seite hin aus dem Sprühkopf ausstossen. Die beiden Komponenten, die aus den Komponentenauslässen 8 und 8' ausgetragen werden, werden somit jeweils in von einander weg weisenden Richtungen ausgetragen. Zwischen den beiden Komponentenauslässen 8 und 8' bilden die gewinkelt angeordneten Flächen 18 und 18' eine Spitze 19 aus, die als Abschirmmittel für die beiden Komponentenauslässe 8 und 8' wirkt. Dadurch, dass die Komponenten schräg zur Seite aus den Komponentenauslässen 8 und 8' ausgetragen werden und auch durch das Abschirmmittel 19 zwischen den Komponentenauslässen kann sichergestellt werden, dass die einzelnen Komponenten nicht unmittelbar nach dem Austreten aus den Komponentenauslässen miteinander in Kontakt treten. Dadurch wird verhindert, dass der Sprühkopf, bzw. die Sprühkopfspritze, durch unerwünschten vorzeitigen Kontakt der Komponenten verklebt und ein Versprühen der Komponenten nicht mehr möglich ist. In der dargestellten Ausführungsform, weisen die Flächen 18, 18' ca. einen 90 Grad Winkel zueinander auf. Die Komponentenauslässe 8 und 8' sind daher in einem 45 Grad Winkel zur Längsachse der Komponentenkanäle 6 und 6' ausgerichtet. Andere Winkel sind möglich, solange die beschriebene Funktion der gewinkelten Flächen erfüllt wird.

In Figur 4b ist die Sprühkopfspitze 7 in einer Schnittebene gezeigt, die durch zwei sich gegenüberliegenden Druckgasauslasskanälen 17, 17' verläuft, die zwischen den Stegen 16, 16' ausgebildet sind. Die Kappe 10 und das Gehäusebauteil 9 sind im Bereich der Sprühkopfspitze 7 konisch in Richtung der Spitze zulaufend ausgebildet. Die Längsrichtung der Druckgasauslasskanäle 17 und 17' ist somit schräg zur Längsachse des Gehäusebauteils 9, bzw. der Komponentenkanäle 6 und 6' ausgerichtet. Die Druckgasauslasskanäle 17 und 17' sind daher auf einen Punkt vor der Sprühkopfspitze 7 fokussiert.

Aus den Figuren 4a und 4b ist zu entnehmen, dass die Druckgasauslasskanäle 17 und 17' nicht in der Verbindungslinie der beiden Komponentenauslässe 8 und 8' liegen. Entlang der Verbindungslinie der Komponentenauslässe 8 und 8' sind die Stege 16 und 16' vorgesehen. Die Druckgasauslasskanäle 17, 17' sind daher nicht auf die Flächen 18, 18' gerichtet, welche die Komponentenauslässe 8 und 8' aufweisen, sondern sind neben der Verbindungslinie, bzw. den Flächen, angeordnet. Durch eine derartige Anordnung der Druckgasauslasskanäle zu den Komponentenauslässen wird erreicht, dass an den Komponentenauslässen 8 und 8' kein Vakuumeffekt auftritt, durch welchen die Komponenten unkontrolliert aus den Komponentenauslässen gesogen werden könnten. Der Druckgasstrom wird seitlich an den Flächen 18 und 18' vorbeigeführt, so dass das Druckgas erst auf die Komponenten trifft, wenn diese bereits aus den Komponentenauslässen 8 und 8' ausgetreten sind. Da die Druckgasauslasskanäle 17, 17' auf einen Punkt über der Sprühkopfspitze fokussiert sind, werden die Komponenten in diesem Bereich zerstäubt und miteinander vermischt. Durch die Fokussierung der Druckgasauslasskanäle 17, 17' wird auch erreicht, dass die vermischten Komponenten gezielt auf eine Anwendungsstelle aufgetragen werden können. Es entsteht kein breiter Sprühkegel, wie bei herkömmlichen Sprühvorrichtungen.

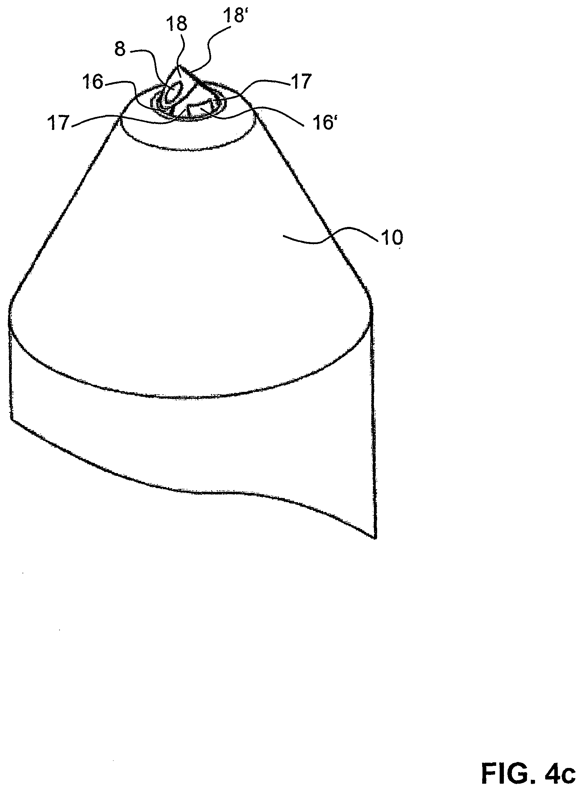

In Figur 4c ist eine dreidimensionale Darstellung der Sprühkopfspitze 7 nach der Erfindung gezeigt. Aus Figur 4c ist ersichtlich, dass die Druckgasauslasskanäle 17, 17' zwischen den Stegen 16, 16' ausgebildet sind und neben, bzw. seitlich der Verbindungslinie der Komponentenauslässe 8, 8', bzw. der Flächen 18, 18' angeordnet sind. Es ist ersichtlich, dass die Komponentenauslässe 8 und 8' ausserhalb der Kappe 10 aus dem Gehäusebauteil 9 münden. Bei dieser Ausführungsform sind auf dem Umfang des Gehäusebauteils 9 vier Stege 16, 16' ausgebildet. Die Stege sind auf der Verbindungslinie der Komponentenauslässe 8 und 8' und auf der Schnittlinie der Flächen 18, 18' vorgesehen. Es bilden sich daher Druckauslasskanäle 17, 17' jeweils zu beiden Seiten einer der Flächen 18 und 18' aus. Das heisst, es sind jeweils zwei Druckgasauslasskanäle 17, 17' auf den beiden Seiten der Komponentenauslässe vorgesehen.

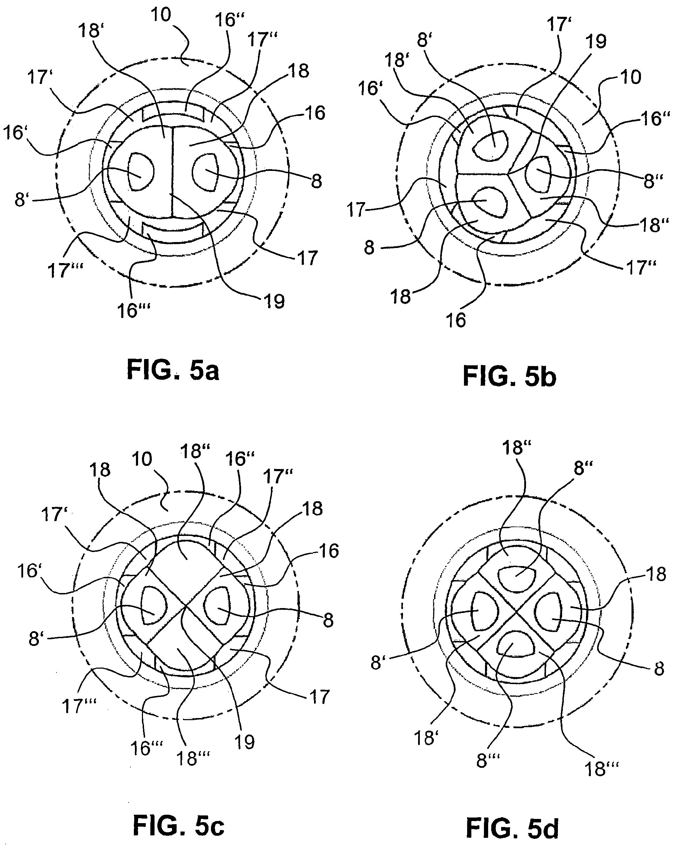

In den Figuren 5a bis 5d sind Frontansichten verschiedener Ausführungsformen von Sprühkopfspitzen gemäss der vorliegenden Erfindung gezeigt. Die Sprühkopfspitzen unterscheiden sich in der Anzahl der Komponentenauslässe und in der Anzahl der schrägen Flächen, aus welchen die Komponentenauslässe münden. In Figur 5a ist eine Sprühkopfspitze gemäss der Ausführungsform der Figuren 4a bis 4c gezeigt. Es sind zwei Komponentenauslässe 8 und 8' an zwei nebeneinander angeordneten Flächen 18 und 18' gezeigt. Die Flächen 18 und 18' sind derart schräg zur Längsachse des Sprühkopfes angeordnet, dass sie zwischen sich eine Kante bilden, die als Abschirmmittel 19 fungiert. Die Sprühkopfspitze ragt derart aus der Kappe 10 hervor, dass sich zwischen den Stegen 16, 16', 16" und 16"' vier Druckgasauslasskanäle 17, 17', 17" und 17"' bilden. Die Druckgasauslasskanäle sind ringförmig um die Komponentenauslässe 8 und 8' angeordnet. Zwei Stege 16 und 16' sind gegenüber den Komponentenauslässen 8 und 8' derart angeordnet, dass sie auf einer Verbindungslinie der Komponentenauslässe 8 und 8' zu liegen kommen. Die Stege 16" und 16"' sind neben dieser Verbindungslinie, bzw. auf einer Linie senkrecht zu dieser angeordnet.

In Figur 5b ist eine Sprühkopfspitze mit drei Komponentenauslässen 8, 8' und 8" gezeigt, die aus drei zueinander schräg angeordneten Flächen 18, 18' und 18" münden. Die Komponentenauslässen 8, 8' und 8" sind dreieckförmig angeordnet. Die drei Stege 16, 16' und 16" bilden zwischen sich drei Druckgasauslasskanäle 17, 17' und 17" aus. Die Stege kommen unterhalb der Flächen 18, 18' und 18" zu liegen. Die Schnittlinien der Flächen 18, 18' und 18" treffen sich im Mittelpunkt der Sprühkopfspitze und bilden so ein spitzenartiges Abschirmmittel 19 aus.

In den Figuren 5c und 5d sind vier Flächen 18, 18', 18" und 18'" vorgesehen, die schräg zur Längsachse des Sprühkopfes verlaufen und deren Schnittlinien sich in einem Punkt treffen. In Figur 5c sind zwei Komponentenauslässe 8 und 8' vorgesehen, die aus zwei sich gegenüberliegenden Flächen 18 und 18' münden. In Figur 5d sind vier Komponentenauslässe 8, 8', 8" und 8'" vorgesehen, die jeweils aus einer der Flächen 18, 18', 18" und 18"' münden.

In den dargestellten Ausführungsbeispielen sind die Flächen, aus welchen die Komponentenauslässe münden, symmetrisch angeordnet und ihre Flächennormalen schneiden mit der Längsachse des Sprühkopfes. Grundsätzlich können die Flächen aber auch asymmetrisch vorgesehen sein, ihre Flächennormalen können neben der Längsachse des Sprühkopfes verlaufen und ihre Flächen können auch gewölbt oder gewunden sein. Wesentlich ist dabei, dass die Komponentenauslässe schräg nach aussen gerichtet sind und vorzugsweise die Flächen zwischen sich ein Abschirmmittel ausbilden.

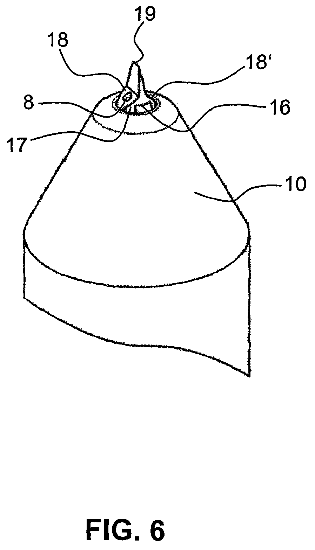

In Figur 6 ist ein weiteres Ausführungsbeispiel einer Sprühkopfspitze mit einem langgestreckten Abschirmmittel 19 gezeigt. Das Abschirmmittel 19 steht plattenartig zwischen den Flächen 18 und 18' hervor. Es ragt im Wesentlichen von der Verbindungslinie, die durch die sich schneidenden Flächen 18 und 18' gebildet wird, entlang der Längsachse des Sprühkopfes von der Sprühkopfspitze nach vorne ab. Ein solches Abschirmmittel verhindert zusätzlich zu den schräg angeordneten Komponentenauslässen ein unerwünschtes vorzeitiges Mischen verschiedener Komponenten und verhindert so ein Verkleben des Sprühkopfes.

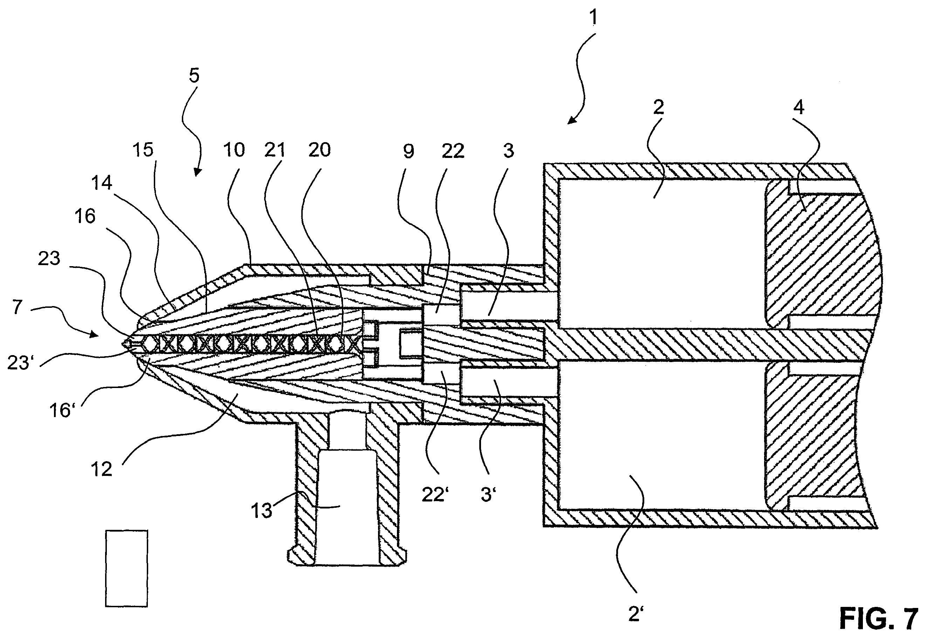

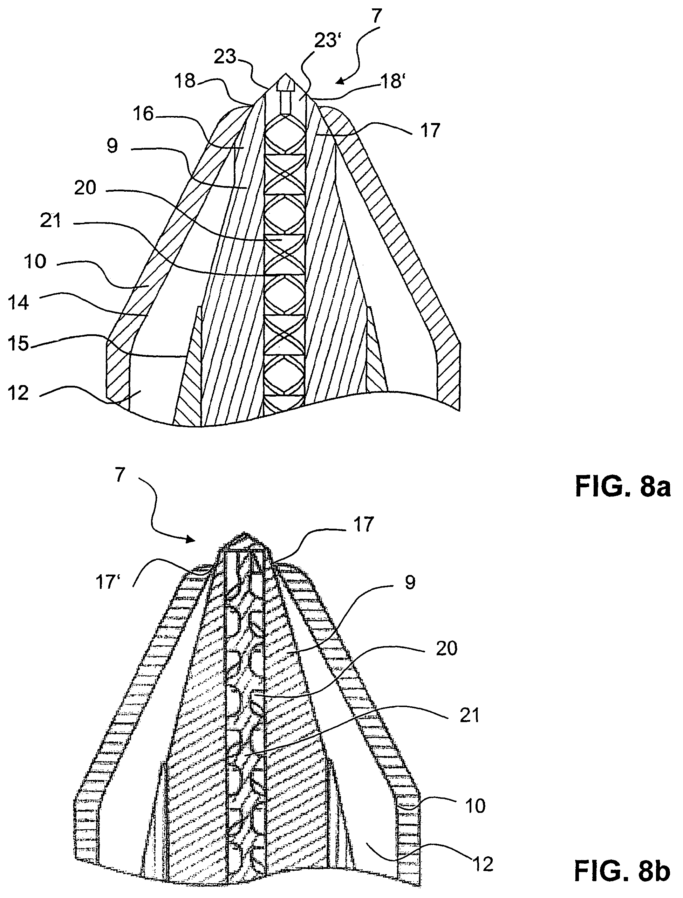

In den Figuren 7 und 8a bis c ist eine weitere Ausführungsform eines Sprühkopfes und einer Sprühvorrichtung nach der vorliegenden Erfindung gezeigt. Bauteile die mit den Bauteilen gemäss der Ausführungsform nach den Figuren 1 bis 4 übereinstimmen, tragen die gleichen Bezugszeichen. In Figur 7 ist der Sprühkopf 5 und ein Abschnitt der Doppelspritze 1 gezeigt. Die Doppelspritze 1 weist zwei Behälter 2 und 2' auf, an die jeweils ein Behälterauslass 3 und 3' angeschlossen ist. In den Behältern 2 und 2' ist ein Doppelkolben 4 als Austragvorrichtung zum Austragen der Komponenten aus den Behältern 2 und 2' vorgesehen.

Bei dieser Ausführungsform weist das Gehäusebauteil 9 einen Komponentenkanal 20 auf, der als Mischkanal dient und in dem Mischelemente 21 angeordnet sind. Das Gehäuse weist weiter zwei Zugangskanäle 22 und 22' auf, die die Behälterauslässe 3, 3' mit dem Komponentenkanal 20 verbinden, wenn das Gehäusebauteil 9 über den Behälterauslässen 3 und 3' der Doppelspritze 1 aufgesetzt ist. Werden die beiden Komponenten aus den Behältern 2 und 2' mittels dem Doppelkolben 4 durch die Behälterauslässe 3, 3' ausgetragen, kommen diese in dem Komponentenkanal 20 in Kontakt und werden durch ein weiteres Einschieben des Doppelkolbens 4 in die Behälter 2 und 2' innerhalb des Komponentenkanals 20 entlang der Mischelement 21 vorgeschoben, wodurch auf Grund der Mischelemente 21 eine Mischung der beiden Komponenten innerhalb des Sprühkopfes erfolgt. Diese Ausführungsform ist daher für das Versprühen von Substanzen geeignet, die kurz vor ihrer Anwendung vermischt werden müssen, jedoch nicht derart schnell aushärten, dass sie den Komponentenkanal, bzw. die Mischelemente verkleben.

Über dem Gehäusebauteil 9 ist die Kappe 10 mit dem Druckgaszufuhrkanal 13 aufgesetzt, so dass sich zwischen dem Gehäusebauteil 9 und der Kappe 10 der Ringkanal 12 ausbildet. An dem Gehäusebauteil 9 sind im Bereich der Sprühkopfspitze 7 Stege 16 und 16' am Aussenumfang angeordnet. Die Stege 16, 16' liegen an der Innenwand 14 der Kappe 10 an. Der Komponentenkanal 20 wird im Bereich seines Ausgangs durch die Mischelemente 21 derart geteilt, dass sich zwei nebeneinander angeordnete Komponentenauslässe 23 und 23' bilden. Beim Austragen der Mischung aus dem Komponentenkanal 20 wird die Mischung daher durch zwei getrennte, dicht nebeneinander liegende Komponentenauslässe 23, 23' ausgestossen.

In Figur 8a ist ein Längsschnitt durch die Sprühkopfspitze 7 gezeigt, der sich durch zwei gegenüberliegende Stege 16, 16' erstreckt. Der Komponentenkanal 20 mit den Mischelementen 21 ist innerhalb des Gebäudebauteils 9 in längs Längsrichtung ausgebildet. Zwischen der Innenwand 14 der Kappe 10 und der Aussenwand 15 des Gehäusebauteils 9 ist der Ringkanal 12 ausgebildet, der im Bereich der Spitze der Sprühkopfspitze 7 durch die Stege 16, 16' in dieser Schnittebene verschlossen ist.

Die Komponentenauslässe 23 und 23' sind an zwei zueinander gewinkelt angeordneten Flächen 18 und 18' angeordnet. Die Funktion der schrägen Flächen 18 und 18' ist die gleiche, wie bei dem vorhergehenden Ausführungsbeispiel.

In Figur 8b ist ein Längsschnitt durch die Sprühkopfspitze 7 entlang einer Ebene zwischen zwei Stegen 16 und 16' dargestellt. Im Inneren des Gehäusebauteils 9 ist der Komponentenkanal 20 mit den Mischelementen 21 gezeigt, der in dieser Schnittebene keinen Auslass aufweist. Zwischen dem Gehäusebauteil 9 und der Kappe 10 ist der Ringkanal 12 ausgebildet, der im Bereich der Sprühkopfspitze 7 in die Druckgasauslasskanäle 17 und 17' mündet.

Die Sprühkopfspitze 7 entspricht in der dreidimensionalen Darstellung der in Figur 4c gezeigten Spitze. Daraus ist ersichtlich, dass im Bereich der Sprühkopfspritze das Gehäusebauteil durch die Öffnung 11 aus der Kappe 10 hervorsteht, so dass die Komponentenauslässe 23 und 23'sowie die Auslässe 8 und 8'in Figur 4c ausserhalb der Kappe 10 zu liegen kommen. Am Aussenumfang des Gehäusebauteils 9 sind die Stege 16, 16' erkennbar. Zwischen den Stegen 16, 16' sind die Druckgasauslasskanäle 17, 17' angeordnet. Die Flächen 18, 18' sind zueinander gewinkelt angeordnet und bilden zwischen den Komponentenauslässen 23, 23' ein Abschirmmittel 19.

Die Ausgestaltung der Druckgasleitung gemäss der vorliegenden Erfindung mit einem Ringkanal und darin angeordneten Stegen zur Kontrolle und Fokussierung des Druckgasausstosses an der Sprühkopfspitze der Sprühvorrichtung entspricht demjenigen des Ausführungsbeispiels gemäss den Figuren 1 bis 4. Zum einen kann durch die Ausbildung der Stege in dem Ringkanal die Menge des zum Versprühen der Komponenten benötigten Druckgases reduziert werden, der Druckgasstrom kann gezielt fokussiert werden und in ausgewählter Weise relativ zu den Komponentenauslässen angeordnet werden. Zum anderen wird durch die Anordnung der Komponentenauslässe an unterschiedlichen Flächen, die zueinander gewinkelt angeordnet sind, ein unkontrolliertes Mischen der ausgetragenen Komponenten verhindert und die negativen Effekte einer Vakuumbildung beim Vorbeiströmen des Druckgases werden reduziert.

In der Beschreibung der Erfindung soll unter Sprührichtung diejenige Richtung verstanden werden, die in der Regel der Längsachse der Sprühvorrichtung entspricht. Es ist jedoch auch möglich, dass eine Substanz in einem Winkel zur Längsachse der Sprühvorrichtung versprüht wird. In diesem Fall kann die Normale, die senkrecht zu einer Auslassöffnung eines erfindungsgemässen Ringkanals verläuft, die Sprührichtung angeben, da ein Druckgasstrom, der sich aus der Summe der aus allen Druckgasauslasskanälen austretenden Ströme zusammensetzt, in diese Richtung verläuft.

### Bezugszeichenliste

- 1: Doppelspritze
- 2, 2': Behälter
- 3, 3': Behälterauslässe
- 4: Doppelkolben
- 5: Sprühkopf
- 6, 6': Komponentenkanäle
- 7: Sprühkopfspitze
- 8, 8': Komponentenauslässe
- 9: Gehäusebauteil
- 10: Kappe
- 11: Öffnung
- 12: Ringkanal
- 13: Druckgaszufuhrkanal
- 14: Kappeninnenwand
- 15: Gehäuseaussenwand
- 16, 16': Stege
- 17, 17': Druckgasauslasskanäle
- 18, 18': Flächen
- 19: Abschirmmittel
- 20: Komponentenkanal
- 21: Mischelemente
- 22, 22': Zugangskanal
- 23, 23': Komponentenauslässe

## Patentansprüche

1. Sprühkopf für eine Sprühvorrichtung zum Versprühen wenigstens einer Substanz, bzw. einer Komponente, der umfasst:
- wenigstens einen Substanz-, bzw. Komponentenkanal (6, 6'; 20) mit wenigstens einem Komponentenauslass (8, 8'; 23, 23'), der aus einer Spitze des Sprühkopfes (7) mündet,
- einen Ringkanal (12) für ein Druckgas, der den wenigstens einen Komponentenkanal (6, 6; 20) in Längsrichtung zumindest teilweise umgibt und an der Spitze des Sprühkopfes (7) aus dem Sprühkopf (5) mündet, und
- einen Druckgaszufuhrkanal (13) zum Einleiten von Druckgas in den Ringkanal (12),
wobei der Ringkanal (12) mehrere Stege (16, 16', 16", 16"') aufweist, die den Ringkanal (12) wenigstens im Bereich der Spitze des Sprühkopfes (7) in voneinander getrennte Druckgasauslasskanäle (17, 17', 17", 17"') unterteilen,
**dadurch gekennzeichnet, dass** entweder
(a) wenigstens zwei Komponentenkanäle (6, 6') für jeweils unterschiedliche Komponenten vorgesehen sind, deren Komponentenauslässe (8, 8') nebeneinander aus der Spitze des Sprühkopfes (7) münden; oder dass
(b) ein einziger Substanz- oder Mischkanal (20) vorgesehen ist, der sich an der Spitze des Sprühkopfes (7) in zwei nebeneinander angeordnete Komponentenauslässe (23, 23') teilt.

2. Sprühkopf nach Anspruch 1, **dadurch gekennzeichnet, dass** die Stege (16, 16') in Richtung der Sprühkopfspitze (7) derart angeordnet sind, dass sich die Druckgasauslasskanäle (17, 17', 17", 17"') in Richtung der Sprühkopfspitze (7) verjüngen.

3. Sprühkopf nach einem der vorhergehenden Ansprüche, **dadurch gekennzeichnet, dass** die Komponentenkanäle (6, 6') bzw. der Substanz- oder Mischkanal (20) in einem Gehäuse (9) ausgebildet sind und der Ringkanal (12) zwischen einer Aussenwand (15) des Gehäuses und einer Innenwand (14) einer auf das Gehäuse aufgesetzten Kappe (10) ausgebildet ist, wobei die Kappe (10) an einem Ende dicht mit dem Gehäuse (9) abschliesst und am anderen Ende eine Öffnung (11) aufweist.

4. Sprühkopf nach Anspruch 3, **dadurch gekennzeichnet, dass** der Druckgaszufuhrkanal (13) an der Kappe (10) vorgesehen ist.

5. Sprühkopf nach Anspruch 3 oder 4, **dadurch gekennzeichnet, dass** die Stege (16, 16', 16", 16"') an der Innenwand (14) der Kappe und / oder der Aussenwand (15) des Gehäuses (9) radial abstehend angeordnet sind.

6. Sprühkopf nach einem der vorhergehenden Ansprüche, **dadurch gekennzeichnet, dass** zwischen zwei Komponentenauslässen (8, 8'; 23, 23') ein Abschirmmittel (19) angeordnet ist.

7. Sprühkopf nach einem der vorhergehenden Ansprüche, **dadurch gekennzeichnet, dass** die Komponentenauslässe (8, 8'; 23, 23') an der Sprühkopfspitze (7) in Sprührichtung über die Druckgasauslasskanäle (17, 17', 17", 17"') hervorstehen.

8. Sprühkopf nach einem der vorhergehenden Ansprüche, **dadurch gekennzeichnet, dass** zwei Komponentenauslässe (8, 8'; 23, 23') vorgesehen sind, die jeweils an einer Fläche (18, 18') der Sprühkopfspitze (7) austreten, wobei die Flächen (18, 18') zueinander einen Winkel derart einschliessen, dass die Komponentenauslässe (8, 8'; 23, 23') schräg zur Sprührichtung nach aussen gerichtet sind.

9. Sprühkopf nach Anspruch 8, **dadurch gekennzeichnet, dass** die Stege (16, 16') derart angeordnet sind, dass die Druckgasauslasskanäle (17, 17', 17", 17"') neben einer Verbindungslinie von zwei Komponentenauslässen (8, 8'; 23, 23') angeordnet sind.

10. Sprühkopf nach Anspruch 9, **dadurch gekennzeichnet, dass** zwei der Stege (16, 16') auf einer Verbindungslinie von zwei Komponentenauslässen (8, 8'; 23, 23') angeordnet sind, derart, dass Druckgas nicht direkt über die Komponentenauslässe strömt.

11. Sprühkopf nach Anspruch 10, **dadurch gekennzeichnet, dass** sich die Flächen (18, 18') an einer Schnittlinie treffen, und dass zwei der Stege (16", 16"') beidseitig entlang der Schnittlinie angeordnet sind, so dass für jede der Flächen (18, 18') die Druckgasauslasskanäle (17, 17') zu beiden Seiten der jeweiligen Fläche (18; 18') ausgebildet sind.

12. Sprühkopf nach einem der Ansprüche 1-7, **dadurch gekennzeichnet,**
**dass** die Sprühkopfspitze mindestens zwei Komponentenauslässe (8, 8', 8") und mindestens zwei Flächen (18, 18', 18", 18"'), die schräg zur Längsachse des Sprühkopfs verlaufen, aufweist, wobei jeweils ein Komponentenauslass aus jeder der Flächen mündet, und
**dass** für jede der Flächen (18, 18', 18", 18"') einer der Stege (16, 16', 16", 16'") unterhalb der betreffenden Fläche angeordnet ist.

13. Sprühvorrichtung mit einem Sprühkopf (5) nach einem der Ansprüche 1 bis 12.

14. Sprühvorrichtung nach Anspruch 13, **dadurch gekennzeichnet, dass** wenigstens zwei Behälter (2, 2') zur Aufnahme jeweils unterschiedlicher Komponenten, die jeweils einen Behälterauslass (3, 3') aufweisen, welche in den wenigstens einen Komponentenkanal (6, 6'; 20) des Sprühkopfes (5) münden, und eine Austragvorrichtung (4) zum Austragen der Komponenten aus den Behältern (2, 2') vorgesehen sind.

15. Sprühvorrichtung nach einem der Ansprüche 13 oder 14, **dadurch gekennzeichnet, dass** die Behälter (2, 2') durch eine Doppelkartusche oder Doppelspritze (1) gebildet werden.

## Claims

1. A spray head for a spraying device for spraying at least one substance or component, comprising:
- at least one substance or component duct (6, 6'; 20), with at least one component outlet (8, 8'; 23, 23') that opens out from a spray head tip (7),
- an annular duct (12) for a pressurized gas, which annular duct (12) at least partially surrounds the at least one component duct (6, 6'; 20) in the longitudinal direction and opens out from the spray head (5) at the spray head tip (7), and
- a pressurized gas supply duct (13) for introducing pressurized gas into the annular duct (12),
wherein the annular duct (12) has a plurality of webs (16, 16', 16", 16"'), which divide the annular duct (12), at least in the area of the spray head tip (7), into pressurized gas outlet ducts (17, 17', 17", 17"') that are separate from one another,
**characterised in that** either
(a) at least two component ducts (6, 6') for different components are provided, the component ducts (6, 6') having component outlets (8, 8') tat open out from the spray head tip (7) alongside each other; or
(b) a single substance or mixing duct (20) is provided, the substance or mixing duct (20) dividing at the spray head tip (7) into two component outlets (23, 23') arranged alongside each other.

2. The spray head as claimed in claim 1, **characterised in that** the webs (16, 16') are arranged in the direction of the spray head tip (7) in such a way that the pressurized gas outlet ducts (17, 17', 17', 17") taper in the direction of the spray head tip (7).

3. The spray head as claimed in any one of the preceding claims, **characterised in that** the component ducts (6, 6') or the substance or mixing duct (20), respectively, are formed in a housing (9), and the annular duct (12) is formed between an outer wall (15) of the housing and an inner wall (14) of a cap (10) fitted onto the housing, wherein the cap (10) at one end is closed tightly with the housing (9) and at the other end has an opening (11).

4. The spray head as claimed in claim 3, **characterised in that** the pressurized gas supply duct (13) is provided on the cap (10).

5. The spray head as claimed in claim 3 or 4, **characterised in that** the webs (16, 16', 16", 16"') are arranged in a radially protruding manner on the inner wall (14) of the cap and/or on the outer wall (15) of the housing (9).

6. The spray head as claimed in any one of the preceding claims, **characterised in that** a screening means (19) is arranged between two component outlets (8, 8'; 23, 23').

7. The spray head as claimed in any one of the preceding claims, **characterised in that** the component outlets (8, 8'; 23, 23') on the spray head tip (7) protrude beyond the pressurized gas outlet ducts (17, 17', 17", 17"') in the spraying direction.

8. The spray head as claimed in any one of the preceding claims, **characterised in that** two component outlets (8, 8'; 23, 23') are provided which each emerge on a surface (18, 18') of the spray head tip (7), wherein the surfaces (18, 18') enclose an angle between each other such that the component outlets (8, 8'; 23, 23') are directed outward at an inclination with respect to the spraying direction.

9. The spray head as claimed in claim 8, **characterised in that** the webs (16, 16') are arranged in such a way that the pressurized gas outlet ducts (17, 17', 17", 17"') are arranged next to a connecting line of two component outlets (8, 8'; 23, 23').

10. The spray head as claimed in claim 9, **characterised in that** two of the webs (16, 16') are arranged on a connecting line of two component outlets (8, 8'; 23, 23') in such a manner that pressurized gas does not flow directly over the component outlets.

11. The spray head as claimed in claim 10, **characterised in that** the surfaces (18, 18') meet at a connecting line, and **in that** two of said webs (16", 16"') are arranged on both sides along the connecting line of the surfaces (18, 18'), in such a manner that for each of the surfaces (18, 18') the pressurized gas outlet ducts (17, 17') are disposed on both sides of the respective surface (18, 18').

12. The spray head as claimed in any one of claims 1-7, **characterised in**
**that** the spray head tip comprises at least two component outlets (8, 8', 8") and at least two surfaces (18, 18', 18", 18"') which extend obliquely to the longitudinal axis of the spray head, one component outlet opening out from each of the surfaces, and
**that** for each of the surfaces (18, 18', 18", 18"') one of the webs (16, 16', 16", 16"') is arranged below the respective surface.

13. A spraying device comprising a spray head (5) as claimed in any one of claims 1 through 12.

14. The spraying device as claimed in claim 13, **characterised in that** at least two containers (2, 2') are provided for receiving different components, which containers (2, 2') have respective container outlets (3, 3'), which open into the at least one component duct (6, 6'; 20) of the spray head (5), and a discharge device (4) for discharging the components from the containers (2, 2').

15. The spraying device as claimed in either of claims 13 and 14, **characterised in that** the containers (2, 2') are formed by a double cartridge or double syringe (1).

## Revendications

1. Une tête de pulvérisation pour dispositif de pulvérisation pour pulvériser au moins une substance resp. un composant qui comprend :
- au moins un canal à substance resp. à composant (6, 6'; 20) ayant au moins une sortie à composant (8, 8'; 23, 23') débouchant d'une pointe (7) de la tête de pulvérisation,
- un canal annulaire (12) pour un gaz sous pression entourant au moins en partie le canal à composant (6, 6'; 20) en direction longitudinale et débouchant de la tête de pulvérisation (5) à la pointe (7) de la tête de pulvérisation, et
- un canal d'alimentation (13) en gaz sous pression pour introduire du gaz sous pression dans le canal annulaire (12),
dans laquelle le canal annulaire (12) présente plusieurs nervures (16, 16', 16", 16"') qui divisent le canal annulaire (12), au moins dans la région de la pointe (7) de la tête de pulvérisation, en canaux de sortie du gaz sous pression (17, 17', 17", 17'") séparés les uns des autres, **caractérisée en ce que**, ou bien
(a) au moins deux canaux à composant (6, 6') sont prévus pour des composantes respectivement différentes, dont les sorties à composant (8, 8') débouchent l'une à côté de l'autre de la pointe (7) de la tête de pulvérisation, ou bien
(b) un canal unique à substance resp. de mélange (20) est prévu, qui se divise en deux sorties à composant (23, 23'), arrangées l'une à côté de l'autre, à la pointe (7) de la tête de pulvérisation.

2. La tête de pulvérisation selon la revendication 1, **caractérisée en ce que** les nervures (16, 16') sont arrangées en direction de la pointe (7) de la tête de pulvérisation tel que les canaux de sortie du gaz sous pression (17, 17', 17" , 17"') se rétrécissent en direction de la pointe (7) de la tête de pulvérisation.

3. Tête de pulvérisation selon une des revendications précédentes, **caractérisée en ce que** les canaux à composant (6, 6'), respectivement le canal à substance ou de mélange (20) sont formés dans un boîtier (9) et le canal annulaire (12) est formé entre une paroi extérieure (15) du boîtier et une paroi intérieure (14) d'un capuchon (10) posé sur le boîtier, où le capuchon (10) est monté de manière étanche à une extrémité avec le boîtier (9) et présente sur l'autre extrémité une ouverture (11).

4. Tête de pulvérisation selon la revendication 3, **caractérisée en ce que** le canal d'alimentation en gaz sous pression (13) est prévu sur le capuchon (10).

5. Tête de pulvérisation selon la revendication 3 ou 4, **caractérisée en ce que** les nervures (16, 16', 16", 16"') sont arrangées sur la paroi intérieure (14) du capuchon et/ou la paroi extérieure (15) du boîtier (9) de manière saillante en direction radiale.

6. Tête de pulvérisation selon une des revendications précédentes, **caractérisée en ce que** un moyen d'isolation (19) est arrangé entre deux sorties à composant (8, 8', 23, 23').

7. Tête de pulvérisation selon une des revendications précédentes, **caractérisée en ce que** les sorties à composant (8, 8', 23, 23') sont arrangées sur la pointe (7) de la tête de pulvérisation s'étendant en direction de pulvérisation au-delà des canaux de sortie du gaz sous pression (17, 17', 17", 17"').

8. Tête de pulvérisation selon une des revendications précédentes, **caractérisé en ce que** deux sorties à composant (8, 8', 23, 23') sont prévues, qui débouchent respectivement sur une surface (18, 18') de la pointe (7) de tête de pulvérisation, ou les surfaces (18, 18') renferment l'une par rapport à l'autre un angle tel que les sorties à composant (8, 8', 23, 23') sont orientées vers l'extérieur et de manière décalée par rapport à la direction de pulvérisation.

9. Tête de pulvérisation selon la revendication 8, **caractérisée en ce que** les nervures (16, 16') sont arrangés tel que les canaux de sortie du gaz sous pression (17, 17', 17", 17"') sont arrangés à côté d'une ligne de connexion de deux sorties à composant (8, 8', 23, 23').

10. Tête de pulvérisation selon la revendication 9, **caractérisée en ce que** deux des nervures (16, 16') sont arrangés sur une ligne de connexion de deux sorties à composant (8, 8', 23, 23'), tel que le gaz sous pression ne s'écoule pas directement sur les sorties à composant.

11. Tête de pulvérisation selon la revendication 10, **caractérisée en ce que** les surfaces (18, 18') se rencontrent en une ligne de d'intersection, et que deux des nervures (16", 16"') sont arrangés d'un côté et d'autre le long de la ligne d'intersection, tel que pour chacune des surfaces (18, 18') les canaux de sortie du gaz sous pression (17, 17') sont formés des deux côtés de la surface correspondante (18, 18').

12. Tête de pulvérisation selon une des revendications 1 à 7, **caractérisée en ce que** la pointe de tête de pulvérisation présente au moins deux sorties à composant (8, 8', 8") et au moins deux surfaces (18, 18', 18", 18"') qui s'étendent de manière inclinée à l'axe longitudinal de la tête de pulvérisation, où respectivement une sortie à composant débouche de chacune des surfaces, et que pour chacune des surfaces (18, 18', 18" , 18"') une des nervures (16, 16', 16", 16'") est arrangée en dessous de la surface correspondante.

13. Dispositif de pulvérisation ayant une tête de pulvérisation (5) selon une des revendications 1 à 12.

14. Dispositif de pulvérisation selon la revendication 13, **caractérisé en ce que** au moins deux réservoirs (2, 2') sont prévus pour recevoir respectivement des composants différents, qui respectivement présentent une sortie de réservoir (3, 3'), qui débouchent dans au moins un canal à composant (16, 16' ; 20) de la tête de pulvérisation (5) et un dispositif de décharge (4) pour décharger les composants hors du récipient (2, 2').

15. Dispositif de pulvérisation selon une des revendications 13 ou 14, **caractérisé en ce que** le récipient (2, 2') est formé par une cartouche double ou double seringue (1).
